# EUROPÄISCHE PATENTSCHRIFT

(11) **EP 0 594 926 B1**
(45) Veröffentlichungstag und Bekanntmachung des Hinweises auf die Patenterteilung: **07.01.1998**
(21) Anmeldenummer: 92890231.1
(22) Anmeldetag: 30.10.1992
(51) Int. Cl.: A61K 7/04, A61K 7/043

(54) **Mittel zur Härtung von hornartigen Körperteilen von Säugetieren**
Preparation for hardening the horny tissues of mammalians
Composition durcissante pour les tissus cornés des mammifères

(43) Veröffentlichungstag der Anmeldung: 04.05.1994
(73) Patentinhaber: Haerpfer-Horn, Herta, Mag., A-1040 Wien (AT)
(72) Erfinder: Haerpfer-Horn, Herta, Mag., A-1040 Wien (AT)
(74) Vertreter: Itze, Peter, Dipl.-Ing.

(56) Entgegenhaltungen:
- FR-A- 1 526 996
- GB-A- 2 129 298
- US-A- 3 510 554

## Beschreibung

Die Erfindung bezieht sich auf eine Verwendung von Zitronenöl in einem Verfahren zur Herstellung eines Mittels zur Härtung von homartigen Körperteilen von Säugetieren, insbesondere der Hufe von Pferden, sowie auf das entsprechend hergestellte Mittel.

Die FR-A- 1 526 996 offenbart ein Hauptflegemittel, das ein Gemisch aus ätherischen Ölen, welches als untergeordneten Bestandteil auch Zitronenöl enthält, in niedriger Dosierung (etwa 3 %) enthält, wobei der Rest des Mittels aus reinen pflanzlichen Ölen besteht. Das Zitronenöl wirkt dabei gegen Falten und hat astringierende Wirkung, wobei das Öl lubrifizierend auf die Haut wirkt.

Um hornartige Körperteile von Säugetieren, also z.B. Fingernägel, Krallen, u.dgl., zu pflegen und zu regenerieren, insbesondere aber um einem Pferd gesunde, d.h. feste und doch elastische Hufe zu erhalten, muß der Huf - der Begriff "Huf" wird in diesem Zusammenhang im engeren Sinn als der von der Oberhaut gebildete Hornüberzug des Gliedmaßenendes des Huftieres verstanden - (beim Rind: Klaue) sowohl vor zuviel Nässe, wie auch vor zu starkem Austrocknen geschützt werden. Wird dies verabsäumt, so kann es durch die Einwirkung von zuviel Feuchtigkeit auf den Huf zu einer Huferweichung kommen, die im Extremfall zu einem völligen Verlust der Schutzfunktion des Hufes im eigentlichen Sinne und damit zur Gehunfähigkeit des Pferdes führen kann. Wird hingegen der Huf nicht durch Feuchthaltung und Einfettung vor zu starkem Austrocknen geschützt, wird dieser spröde, rissig und brüchig; es kommt zu Absplitterungen und Wegbruch von Hufteilen am Tragerand (dem unteren Teil des Hufes) und damit zu einer Beschädigung der Befestigungsgrundlage der Hufeisen, wodurch sich dieses oft vom Pferdefuß löst und einen neuen Beschlag erforderlich macht. Ein ausgetrockneter Huf kann aber auch Ursache für diverse leichtere und schwerere Huferkrankungen sein (Hornriß, Hornspalte, Steingalle u.a.m.), deren Ausheilung mit langen Stehzeiten des Pferdes verbunden sind.

Die im Handel erhältlichen Produkte dienen überwiegend zur Erhaltung und Pflege eines guten Hufzustandes, wobei ein Erweichen, Brüchig-, Spröde- oder Rissigwerden der Hufe damit verhindert werden soll. Befindet sich ein Huf jedoch in einem schlechten Zustand, so stehen zu dessen Beseitigung nur wenige Produkte zur Verfügung, deren positive Wirkung jedoch erfahrungsgemäß gering ist. Ein Huf in einem extrem schlechten Zustand kann nur durch dessen Aufbau mittels einer plastischen Hufmasse - ein sehr kostspieliges Verfahren - wieder funktionsfähig gemacht werden.

Der Erfindung liegt die Aufgabe zugrunde, auf rein biologische Weise brüchige, spröde, rissige sowie weiche und mürbe hornartige Körperteile von Säugetieren, insbesondere die Hufe von Pferden, zu behandeln und wiederherzustellen. Insbesondere soll ein Mittel geschaffen werden, welches einerseits einen weichen und mürben Huf härtet und festigt und andererseits bei einem rissigen, spröden und brüchigen Huf den nötigen Grad von Nachgiebigkeit (Elastizität) und Geschmeidigkeit wieder herstellt, der einen einwandfreien Ablauf des Hufmechanismus gewährleistet.

Diese Aufgabe wird erfindungsgemäß dadurch gelöst, daß das Zitronenöl in einem Trägeröl auf pflanzlicher Basis gelöst wird. Dadurch wird ein Mittel erreicht, bei welchem durch die Wirkung des Zitronenöls, das den erfindungsgemäß wichtigen hufhärtenden und -festigenden Wirkstoff enthält, der Härtungseffekt erzielt wird, wobei durch das Trägeröl auf pflanzlicher Basis sowohl das Einbringen des Zitronenöls in die Oberfläche des hornartigen Körperteiles als auch ein Geschmeidighalten dieses Körperteiles erfolgt. Das Trägeröl hat damit einen Doppeleffekt, nämlich zusätzlich zu der bekannten pflegenden Wirkung auch die "Trägerfunktion" zu erfüllen, indem das wirksame Zitronenöl in den Körperteil eingebracht wird. Als Zitronenöl wird vorliegend die Essenz der Frucht des Zitronenbaumes bezeichnet.

Aufgrund der sich in ihren Wirkungen ergänzenden Kombination von ätherischen Ölen und Pflanzenölen ergibt sich durch die Wechselwirkung der Wirkstoffe der Essenzen und der Pflanzenöle eine optimale Wirkungssubstanz. Das weite Wirkungsspektrum der ätherischen Öle kann durch die Verwendung von Pflanzenölen von hoher Qualität als Trägeröle, in denen die Essenzen gelöst werden, weitgehend ausgenützt werden - zusammen mit der hohen Pflegewirkung der den Pflanzenölen innewohnenden Vital- und Pflegestoffe wird daher durch die Anwendung des erfindungsgemäßen Mittels ein optimales Ergebnis in relativ kurzer Zeit erzielt.

Die Härtung und Festigung des Hufes erfolgt durch direktes Auftragen der erfindungsgemäßen Mixtur auf die Hornwand, welche die Ölmischung weitgehend absorbiert; die hufhärtenden Wirkstoffe des ätherischen Zitronenöls können dadurch von innen beginnend eingesetzt werden. Die Verfestigung der Hornwand erfolgt demgemäß von innen nach außen, wodurch es zu einer echten und dauernden Härtung des Hufes kommt.

Vorteilhafterweise können als ätherische Öle zusätzlich noch Salbeiöl und/oder Zypressenöl und/oder Lavendelöl und/oder Tea Tree Öl im Trägeröl auf pflanzlicher Basis gelöst werden. Diese Mittel erhöhen im wesentlichen den pflegenden Charakter des Trägeröls auf pflanzlicher Basis. Es kommt dabei zu besonders wirkungsvollen Kombinationen, da sich die Wirkungen der ätherischen Öle nicht linear addieren, sondern sich potenzieren können, wenn sich ihre Wirkungen ergänzen und gegenseitig unterstützen. Durch das angeführte Hinzufügen von Salbeiöl und/oder Zypressenöl und/oder Lavendelöl und/oder Tea Tree Öl wird nicht nur der Härtungseffekt des Zitronenöls verstärkt, sondern gleichzeitig werden die Hufe vor Fäulnisbildung, Pilzbefall und Infektionen geschützt und auch geglättet. Weiters wird das Wasserbindungsvermögen der Hufe erhöht. Als Trägeröl auf pflanzlicher Basis kann dabei vorteilhafterweise Weizenkeimöl und/oder Mandelöl eingesetzt werden, welche Öle aufgrund ihres Molekülaufbaues ein besonders gutes Eindringen in die zu behandelnden hornartigen Körperteile ergeben.

Um diese optimalen Ergebnisse gewährleisten zu können, werden als Basisöle nur 100%ig reine, kaltgepreßte Pflanzenöle verwendet, die von der Hufoberfläche weitestgehend absorbiert werden. Desgleichen enthält die erfindungsgemäße Mixtur nur reine und echte ätherische Öle, welche mindestens den Qualitätsanforderungen des Österreichischen Arzneibuches (Ö.A.B.) entsprechen.

Die angeführten Öle werden deshalb eingesetzt, weil Weizenkeimöl einen sehr hohen Anteil an Vitamin E enthält, welches - neben anderen Eigenschaften - die bereits sich in der Hornschicht befindlichen Fette vor dem Ranzigwerden schützt. Mandelöl ist das am schnellsten einziehende Öl mit großen pflegenden Eigenschaften.

Bei der Behandlung der Hufe von Pferden wird in drei Stufen vorgegangen, wobei zur Erzielung der Grundhärte der Hufe der Pferde das Verhältnis von ätherischen Ölen zum Trägeröl auf pflanzlicher Basis etwa 3 : 7 betragen kann, wobei der Anteil an ätherischen Ölen wenigstens 70 % Zitronenöl enthält. Bei diesem ersten Mittel überwiegt die Wirkung des Zitronenöls, d.h. daß hier der stärkste Härtungseffekt auftritt, wobei das Trägeröl eben der guten Diffundierung in den Pferdehuf zuträglich ist. In den ätherischen Ölen sind dabei wenigstens 70 % Zitronenöl enthalten, d.h. also, daß auch der Anteil an ätherischen Ölen entsprechend viel Zitronenöl enthält.

Dabei können bei einem entsprechend dem Vorgesagten hergestellten Mittel an ätherischen Ölen wenigstens noch 9 % Salbeiöl und/oder wenigstens 7 % Zypressenöl und/oder wenigstens 8 % Lavendelöl und/oder wenigstens 6 % Tea Tree Öl enthalten sein. Diese Zahlenangaben sind Mindestwerte, wobei innerhalb der gesamten Zahlenangaben der ätherischen Öle natürlich die eingesetzte Menge so variiert wird, daß der Gesamtanteil 100 % nicht überschreitet, d.h. wenn der Anteil an Zitronenöl von 70 % auf 80 % gesetzt wird, dann nehmen die übrigen Anteile entsprechend ab, wobei bei Weglassen eines der Anteile die anderen Anteile so verändert werden, daß der Gesamtanteil 100 % nicht übersteigt. Bei dem in der ersten Stufe angewandten Mittel muß dabei beachtet werden, daß das Mittel nicht auf die Weichteile der Pferdehufe, wie den Strahl bzw. die Knorpel oder den Kronenrand, gelangt, sondern daß eben nur bis etwa 1,5 bis 2 cm an den Kronenrand heran gestrichen wird und nur der wirklich hornartige Teil des Hufes behandelt wird.

In einer zweiten Behandlungsstufe kann das Mittel zur Festigung der Grundhärte der Hufe das Verhältnis von ätherischen Ölen zum Trägeröl auf pflanzlicher Basis etwa 2 : 8 aufweisen, wobei der Anteil an ätherischen Ölen wenigstens 35 % Zitronenöl enthält. Es ist erkennbar, daß in diesem Stadium der Anteil an Zitronenöl am gesamten Mittel erheblich herabgesetzt ist, und daß hier schon mehr die pflegende Wirkung des Trägeröls sowie die Tiefenwirkung des Zitronenöls eingesetzt wird. Dabei kann der Anteil an ätherischen Ölen zusätzlich wenigstens 20 % Salbeiöl und/oder wenigstens 17 % Lavendelöl und/oder wenigstens 16 % Zypressenöl und/oder wenigstens 12 % Tea Tree Öl enthalten. Auch hinsichtlich dieser Zahlenangaben gilt, daß innerhalb der Zusammensetzungen so variiert werden kann, daß, falls eine Komponente verwendet wird, deren Anteil wenigstens den angeführten Wert aufweist, wobei jedoch die Gesamtmenge 100 % nicht überschreiten darf. Bei Wegfall einer Komponente werden dann die anderen anteilsmäßig so variiert, daß insgesamt 100 % erreicht werden.

In einer letzten Stufe kann das Mittel zur Erhaltung und Pflege der verfestigten Grundhärte das Verhältnis von ätherischen Ölen zum Trägeröl auf pflanzlicher Basis etwa 1 : 9 betragen, wobei der Anteil an ätherischen Ölen wenigstens 20 % Zitronenöl enthält. Dieses Mittel dient nur noch der Erhaltung der verfestigten Grundhärte, wobei hier im wesentlichen der pflegende Teil des auf pflanzlicher Basis basierenden Trägeröls zum Tragen kommt. Der Anteil an Zitronenöl dient der Nachhärtung und Pflege der neu hinzugekommenen Hornteile. Bei diesem Mittel kann der Anteil an ätherischen Ölen zusätzlich noch wenigstens 25 % Salbeiöl und/oder wenigstens 25 % Lavendelöl und/oder wenigstens 25 % Zypressenöl und/oder wenigstens 5 % Tea Tree Öl enthalten, was eine besonders gute Geschmeidigkeit und Elastizität des Hufes ergibt. Auch bei dieser Variante gilt hinsichtlich der Obergrenze bei Weglassen einer Komponente das bereits zu den vorhergehenden Zusammensetzungen Gesagte.

Das Mittel, das in der dritten Stufe angewandt wird, kann ohne weiteres auch im Bereich der weichen Hufteile, also des Strahles, der Knorpel und des Kronenrandes, angewandt werden, ohne daß aufgrund der Schärfe des Zitronenöls bzw. anderer Bestandteile die Hautbereiche irritiert werden.

Die Anwendung des Mittels erfolgt dabei zeitmäßig so, daß das Mittel zur Erzielung der Grundhärte der Hufe der Pferde etwa 14 Tage verabreicht wird, wonach dann eine ausreichende Grundhärte vorhanden sein sollte. Wäre dies nicht der Fall, dann kann die Behandlung ohne weiteres noch fortgesetzt werden. Nach Erreichen der Grundhärte wird diese Grundhärte etwa 14 Tage durch Verwendung des Mittels der zweiten Stufe gefestigt. Es hat sich gezeigt, daß nach dieser Behandlung der Huf eine ausreichende Festigkeit und Elastizität für die normale Arbeit mit dem Pferd aufweist. Danach kann dann das Mittel der dritten Stufe, also das Mittel zur Erhaltung und Pflege des Hufes, unbegrenzt lange aufgestrichen werden, ohne daß irgendwelche Beeinträchtigungen aufgrund einer zu langen Gabe erfolgen.

## Patentansprüche

1. Verwendung von Zitronenöl in einem Verfahren zur Herstellung eines Mittels zur Härtung von hornartigen Körperteilen von Säugetieren, insbesondere der Hufe von Pferden, wobei das Zitronenöl in einem Trägeröl auf pflanzlicher Basis gelöst wird.

2. Verwendung nach Anspruch 1, wobei als ätherische Öle zusätzlich Salbeiöl und/oder Zypressenöl und/oder Lavendelöl und/oder Tea Tree Öl im Trägeröl auf pflanzlicher Basis gelöst werden.

3. Verwendung nach Anspruch 1 oder 2, wobei als Trägeröl auf pflanzlicher Basis Weizenkeimöl und/oder Mandelkeimöl eingesetzt wird.

4. Ein gemäß einem der Ansprüche 1 - 3 hergestelltes Mittel, dadurch gekennzeichnet, daß zur Erzielung der Grundhärte der Hufe der Pferde das Verhältnis von ätherischen Ölen zum Trägeröl auf pflanzlicher Basis etwa 3 zu 7 beträgt, wobei der Anteil an ätherischen Ölen wenigstens 70 % Zitronenöl enthält.

5. Mittel nach Anspruch 4, dadurch gekennzeichnet, daß der Anteil an ätherischen Ölen zusätzlich wenigstens 9 % Salbeiöl und/oder wenigstens 7 % Zypressenöl und/oder wenigestens 8 % Lavendelöl und/oder wenigstens 6 % Tea Tree Öl enthält.

6. Mittel nach einem der Ansprüche 1 bis 3, dadurch gekennzeichnet, daß zur Festigung der Grundhärte des Verhältnis von ätherischen Ölen zum Trägeröl auf pflanzlicher Basis etwa 2 zu 8 beträgt, wobei der Anteil an ätherischen Ölen wenistens 35 % Zitronenöl enthält.

7. Mittel nach Anspruch 6, dadurch gekennzeichnet, daß der Anteil an ätherischen Ölen zusätzlich wenigstens 20 % Salbeiöl und/oder wenigstens 17 % Lavendelöl und/oder wenigstens 16 % Zypressenöl und/oder wenigstens 12 % Tea Tree Öl enthält.

8. Mittel nach einem der Ansprüche 1 bis 3, dadurch gekennzeichnet, daß zur Erhaltung und Pflege der verfestigten Grundhärte das Verhältnis von ätherischen Ölen zum Trägeröl auf pflanzlicher Basis etwa 1 zu 9 beträgt, wobei der Anteil an ätherischen Ölen wenigstens 20 % Zitronenöl enthält.

9. Mittel nach Anspruch 8, dadurch gekennzeichnet, daß der Anteil an ätherischen Ölen zusätzlich wenigstens 25 % Salbeiöl und/oder wenigstens 25 % Lavendelöl und/oder wenigstens 25 % Zypressenöl und/oder wenigstens 5 % Tea Tree Öl enthält.

## Claims

1. A use of lemon oil in a method for producing a preparation for hardening the horny tissues of mammals, in particular horse's hoofs, where the lemon oil is dissolved in a vegetable-based carrier oil.

2. A use according to claim 1, where sage oil and/or cypress oil and/or lavender oil and/or tea tree oil as essential oils is/are also dissolved in the vegetable-based carrier oil.

3. A use according to claim 1 or 2, where wheat germ oil and/or almond oil is/are used in the vegetable-based carrier oil.

4. A preparation produced according to one of claims 1-3, characterized in that for achieving the basic hardness of horse's hoofs, the ratio of essential oils to the vegetable-based carrier oil is approximately 3:7, wherein the essential oil portion contains at least 70% lemon oil.

5. A preparation according to claim 4, characterized in that the essential oil portion also contains at least 9% sage oil and/or at least 7% cypress oil and/or at least 8% lavender oil and/or at least 6% tea tree oil.

6. A preparation according to claims 1 to 3, characterized in that to strengthen the basic hardness of the hoof, the ratio of essential oils to the vegetable-based carrier is approximately 2:8, wherein the essential oil portion contains at least 35% lemon oil.

7. A preparation according to claim 6, characterized in that the essential oil portion also contains at least 20% sage oil and/or at least 17% lavender oil and/or at least 16% cypress oil and/or at least 12% tea tree oil.

8. A preparation according to one of claims 1 to 3, characterized in that to preserve and care for the basic hardness of the hoofs the ratio of essential oils to vegetable-based carrier oil is approximately 1:9, wherein the essential oil portion contains at least 20% lemon oil.

9. A preparation according to claim 8, characterized in that the essential oil portion also contains at least 25% sage oil and/or at least 25% lavender oil and/or at least 25% cypress oil and/or at least 5% tea tree oil.

## Revendications

1. Utilisation d'essence de citron dans un procédé destiné à la fabrication d'une composition durcissante pour les tissus cornés des mammifères, notamment les sabots des chevaux, l'essence de citron étant diluée dans une huile support à base végétale.

2. Utilisation selon la revendication 1, selon laquelle on dilue également dans l'huile support à base végétale de l'essence de sauge et/ou de l'essence de cyprès et/ou de l'essence de lavande et/ou de l'essence de Tea Tree comme huile essentielle.

3. Utilisation selon la revendication 1 ou 2, selon laquelle on emploie de l'huile de germe de blé et/ou de l'huile de germe d'amande comme huile support à base végétale.

4. Composition fabriquée selon l'une des revendications 1 à 3 caractérisé en ce que, pour obtenir la dureté de base des sabots de cheval, le rapport entre les huiles essentielles et l'huile support à base végétale est d'à peu près 3 pour 7, la fraction d'huiles essentielles comprenant au moins 70% d'essence de citron.

5. Composition selon la revendication 4 caractérisée en ce que la fraction d'huiles essentielles comprend également au moins 9% d'essence de sauge et/ou au moins 7% d'essence de cyprès et/ou au moins 8% d'essence de lavande et/ou au moins 6% d'essence de Tea Tree .

6. Composition selon l'une des revendications 1 à 3 caractérisée en ce que, pour renforcer la dureté de base, le rapport entre les huiles essentielles et l'huile support à base végétale est d'à peu près 2 pour 8, la fraction d'huiles essentielles comprenant au moins 35% d'essence de citron

7. Composition selon la revendication 6 caractérisée en ce que la fraction d'huiles essentielles comprend également au moins 20% d'essence de sauge et/ou au moins 17% d'essence de lavande et/ou au moins 16% d'essence de cyprès et/ou au moins 12% d'essence de Tea Tree .

8. Composition selon l'une des revendications 1 à 3, caractérisée en ce que, pour maintenir et entretenir la dureté de base renforcée, le rapport entre les huiles essentielles et l'huile support à base végétale est d'à peu près 1 pour 9, la fraction d'huiles essentielles comprenant au moins 20% d'essence de citron.

9. Composition selon la revendication 8 caractérisée en ce que la fraction d'huiles essentielles comprend au moins 25% d'essence de sauge et/ou au moins 25% d'essence de lavande et/ou au moins 25% d'essence de cyprès et/ou au moins 5% d'essence de Tea Tree .
